# EUROPÄISCHE PATENTSCHRIFT

(11) **EP 2 417 094 B1**
(45) Veröffentlichungstag und Bekanntmachung des Hinweises auf die Patenterteilung: **26.12.2012**
(21) Anmeldenummer: 10710548.8
(22) Anmeldetag: 18.03.2010
(51) Int. Cl.: C07C 45/50, C07C 47/02, C07C 45/78, C07C 45/79

(54) **VERFAHREN ZUR AUFARBEITUNG EINES FLÜSSIGEN HYDROFORMYLIERUNGSAUSTRAGS**
METHOD FOR REPROCESSING A LIQUID HYDROFORMYLATION PRODUCT
PROCÉDÉ DE RECYCLAGE D'UN PRODUIT DE SORTIE D'HYDROFORMYLATION LIQUIDE

(30) Priorität: 07.04.2009 DE 102009016652
(43) Veröffentlichungstag der Anmeldung: 15.02.2012
(73) Patentinhaber: OXEA GmbH, 46147 Oberhausen (DE)
(72) Erfinder: GREB, Wolfgang, 46535 Dinslaken (DE); LUKAS, Rainer, 45133 Essen (DE); SCHMID, Klaus, 46539 Dinslaken (DE); ZGORZELSKI, Wolfgang, 46149 Oberhausen (DE); ARNOLD, Jörg, 46535 Dinslaken (DE)
(86) Internationale Anmeldenummer: PCT/EP2010/001719
(87) Internationale Veröffentlichungsnummer: WO 2010/115511

(56) Entgegenhaltungen:
- WO-A1-97/07086
- WO-A2-01/58844

## Beschreibung

Die Erfindung betrifft ein Verfahren zur Aufarbeitung eines flüssigen Hydroformylierungsaustrags einer Hydroformylierungsreaktion, enthaltend mindestens einen Aldehyd als Hydroformylierungsprodukt, nicht umgesetzte olefinisch ungesättigte Verbindungen, gelöstes Synthesegas, den homogen gelösten Hydroformylierungskatalysator sowie Nebenprodukte der Hydroformylierungsreaktion.

Es ist bekannt, dass Verbindungen, die olefinische Doppelbindungen enthalten, mit Kohlenmonoxid und Wasserstoff zu Aldehyden umsetzbar sind (Oxosynthese). Der Prozess ist nicht auf den Einsatz olefinischer Kohlenwasserstoffe begrenzt, sondern erstreckt sich auch auf Ausgangsstoffe, die außer der Doppelbindung noch funktionelle Gruppen aufweisen, vorwiegend solche, die unter den Reaktionsbedingungen unverändert bleiben.

Die klassische Oxosynthese arbeitet mit Kobalt als Katalysator. Seine Wirksamkeit beruht auf der Bildung von Kobaltcarbonylverbindungen unter der Einwirkung von Wasserstoff und Kohlenmonoxid bei Drücken oberhalb 20 MPa und Temperaturen von etwa 120°C und mehr auf metallisches Kobalt oder Kobaltverbindungen.

Im Laufe der Weiterentwicklung der Oxosynthese wurde Kobalt zunehmend durch Rhodium als Katalysatormetall ersetzt. Rhodium wird als Komplexverbindung eingesetzt, das neben Kohlenmonoxid vorzugsweise Phosphine als Liganden enthält. Rhodium als Metall erlaubt es, bei niedrigen Drücken zu arbeiten, überdies erzielt man höhere Ausbeuten und bevorzugt werden die für die Weiterverarbeitung wertvolleren unverzweigten Produkte gebildet, wenn man von geradkettigen endständigen Olefinen ausgeht.

Industriell betrieben wird die Hydroformylierung olefinisch ungesättigter Verbindungen unter der katalytischen Wirkung von Rhodiumcarbonylkomplexen mit tertiären organischen Phosphinen oder Phosphiten als Liganden. Bei einer Prozessvariante arbeitet man in homogener organischer Phase, d.h. eingesetzte olefinisch ungesättigte Verbindung, Katalysator und die Reaktionsprodukte der Hydroformylierungsreaktion liegen gemeinsam in Lösung vor. Die Reaktionsprodukte werden aus dem Gemisch meist destillativ, seltener nach anderen Verfahren wie der Extraktion, abgetrennt. Das in homogener Phase durchgeführte Hydroformylierungsverfahren kann in Form eines Gaskreislaufverfahrens gemäß US 4,247,486 A1 oder in Form eines Flüssigkeitskreislaufsverfahrens gemäß US 4,148,830 A1 ausgestaltet werden.

Als Nebenprodukte bei der Hydroformylierungsreaktion bilden sich hochsiedende Aldehydkondensationsprodukte, bei denen es sich um ein komplexes Gemisch aus sauerstoffhaltigen Verbindungen handelt, die aus den zunächst gebildeten Aldehyden durch Kondensationsreaktionen, wie der Aldolreaktion, oder durch die Tischtschenko-Reaktion entstehen und einen unterschiedlichen Kondensationsgrad aufweisen.

Die Bildung von beispielsweise Dimeren, Trimeren oder Tetrameren als hochsiedende Aldehydkondensationsprodukte bei der homogen durchgeführten Hydroformylierungsreaktion wird beispielsweise in US 4,148,830 A1 beschrieben. Diese hochsiedenden Aldehydkondensationsprodukte eignen sich als Lösungsmittel für den homogen gelösten Rhodiumkatalystor. Zudem stabilisieren sie den Rhodiumkatalysator sowohl in der Hydroformylierungsreaktion als auch bei den nachfolgenden Aufarbeitungsschritten des flüssigen Hydroformylierungsaustrags. Um ihren Anteil in der flüssigen Phase jedoch nicht zu stark ansteigen zu lassen, muss ein Teil stets aus dem Hydroformylierungsprozess entfernt werden. Im stationären Betrieb der Hydroformylierungsanlage werden soviel hochsiedende Aldehydkondensationsprodukte ausgeschleust wie nachgebildet werden.

Bei dem Flüssigkeitskreislaufverfahren gemäß US 4,148,830 A1 wird der flüssige Austrag aus der Hydroformylierungsreaktion zunächst in einem Entspannungsgefäß entspannt, in dem eine Trennung in eine Gasphase und in einer Flüssigphase erfolgt. Die flüssige Phase enthält im Wesentlichen den Katalysator, hochsiedende Aldehydkondensationsprodukte, Lösungsmittel, den gewünschten Aldehyd sowie geringe Mengen nicht umgesetzter olefinisch ungesättigter Verbindung. Die Gasphase enthält überschüssiges Synthesegas, Inerte sowie hydrierte Produkte, geringe Mengen des gebildeten Aldehyds sowie nicht umgesetzte olefinisch ungesättigte Verbindung. Nach Abtrennung und Entfernung eines Anteils der Inerten und hydrierten Produkte wird die Gasphase aufkomprimiert und wieder in den Hydroformylierungsreaktor zurückgeführt. Der flüssige katalysatorhaltige Strom wird auf eine Trenneinrichtung geführt, vorzugsweise auf eine Destillationseinrichtung, bei der als Kopfprodukt die gewünschten Aldehyde sowie geringe Mengen an gelöstem Synthesegas und olefinisch ungesättigter Verbindung entnommen werden. Das Gemisch wird dann in einer weiteren Reinigungsstufe in die reinen n- und iso-Aldehyde aufgetrennt. Der anfallende rhodiumhaltige Stoffstrom, der zudem die hochsiedenden Aldehydkondensationsprodukte als Lösungsmittel enthält, wird in den Hydroformylierungsreaktor zurückgeführt.

WO 01/58844 A2 betrifft die Aufarbeitung eines flüssigen Austrags einer kontinuierlich durchgeführten Hydroformylierungsreaktion. Bei dem bekannten Verfahren erfolgt eine zweistufige Entspannung, wobei in der ersten Stufe auf einen Druck entspannt wird, der 0,2 bis 2 MPa unterhalb des Reaktordruckes liegt. Die dabei anfallende flüssige Phase wird in einer zweiten, niedrigeren Druckstufe weiter entspannt, wobei sich eine Gasphase bildet, die im Wesentlichen die Hauptmenge des gewünschten Hydroformylierungsprodukts enthält. Die dabei anfallende Flüssigphase, die hochsiedende Nebenprodukte und den homogen gelösten Hydroformylierungskatalysator enthält, wird entweder direkt oder nach destillativer Aufarbeitung in den Hydroformylierungsreaktor zurückgeführt.

WO 97/07086 A1 behandelt ebenfalls ein Verfahren zur Aufarbeitung eines flüssigen Hydroformylierungsaustrags, der in einem Entspannungsgefäß neben einer gasförmigen Phase anfällt. Die Flüssigphase wird auf den oberen Teil einer dem Entspannungsgefäß nachgeschaltenen Kolonne gegeben und die gasförmige Phase in den unteren Teil der Kolonne geleitet, so dass die Flüssigphase mit der gasförmigen Phase im Gegenstrom in Kontakt gebracht wird.

Es ist bekannt, dass es bei der destillativen Aufarbeitung des Reaktoraustrags aus der homogen durchgeführten rhodiumkatalysierten Hydroformylierungsreaktion zu einer Zersetzung und/oder Desaktivierung des Rhodiumkatalysators kommen kann. Bei diesen Vorgängen kann es zum einen zur Abscheidung von unlöslichen Rhodiumverbindungen oder Rhodiummetall kommen, die sich in den Apparaturen als Niederschläge oder Beläge festsetzen und nicht mehr in den homogen durchgeführten Hydroformylierungsprozess rezirkuliert werden können. Solche Rhodiummengen stehen für den laufenden Prozess zunächst einmal nicht mehr zur Verfügung und können nur während eines Anlagenstillstands durch gezielte Reinigungsmaßnahmen aus den Apparaturen zurückgewonnen werden. Des Weiteren können durch Zersetzung und Desaktivierung anfallende rhodiumhaltige Feststoffe in dem Destillationsrückstand zwar in suspendierter und damit pumpfähiger Form vorliegen und in den Hydroformylierungsreaktor zurückgeführt werden, doch zeigen solche suspendierte rhodiumhaltige Feststoffe häufig nur noch eine geringe Hydroformylierungsaktivität. Das kann ebenso für Zersetzungs- und Desaktivierungsprodukte gelten, die in dem Destillationsrückstand in gelöster Form vorliegen. Zur Aufrechterhaltung einer vorgegebenen Raum-Zeit-Ausbeute an Aldehyd muss daher mehr Rhodium in Form eines frischen Rhodiumkatalysators zudosiert werden als Rhodium mit den gebildeten hochsiedenden Aldehydkondensationsprodukten entnommen wird. Die Differenz zwischen der zugesetzten Menge an frischem Rhodium und der ausgeschleusten Menge an desaktiviertem Rhodium verbleibt in der Anlage und gilt zunächst als Rhodiumverbrauch.

Es bestand somit die Aufgabe, ein Verfahren zur Aufarbeitung eines flüssigen Austrags einer Hydroformylierungsreaktion bereitzustellen, bei dem die

Zersetzung und/oder Desaktivierung des homogen gelösten Rhodiumkomplexkatalysators wirksam unterdrückt werden kann.

Die Erfindung besteht daher in einem Verfahren zur Aufarbeitung eines flüssigen Austrags einer Hydroformylierungsreaktion, der Aldehyd, hochsiedende Nebenprodukte der Hydroformylierungsreaktion, einen homogen gelösten Rhodiumkomplexkatalysator, nicht umgesetzte olefinisch ungesättigte Verbindung, Synthesegas und leichtflüchtige Nebenprodukte enthält, bei dem
a) der flüssige Hydroformylierungsaustrag in einem Entspannungsbehälter entspannt wird, wobei eine Auftrennung in eine Flüssigphase und in eine Gasphase erfolgt; und
b) die in dem Entspannungsbehälter erhaltene Flüssigphase auf eine Trenneinrichtung gegeben wird, wobei eine Auftrennung erfolgt in eine Flüssigphase, die im Wesentlichen hochsiedende Nebenprodukte der Hydroformylierungsreaktion, den homogen gelösten Rhodiumkomplexkatalysator und geringe Mengen des Aldehyds enthält, und in eine Gasphase, die die überwiegende Menge des Aldehyds enthält; und
c) aus der Trenneinrichtung ein flüssiger, rhodiumhaltiger Austrag abgeführt wird,
dadurch gekennzeichnet, dass der aus der Trenneinrichtung abgeführte flüssige, rhodiumhaltige Austrag über ein Filter geleitet wird und dabei abgetrennte Feststoffe aus dem Verfahren entfernt werden, während das erhaltene Filtrat in die Hydroformylierungsreaktion zurückgeführt wird.

Das erfindungsgemäße Verfahren eignet sich für die Aufarbeitung eines flüssigen, rhodiumhaltigen Austrags aus einer in einem homogenen Reaktionssystem durchgeführten Hydroformylierungsreaktion. Der Begriff homogenes Reaktionssystem steht für eine im Wesentlichen aus Lösungsmittel, Katalysator, olefinisch ungesättigter Verbindung und Reaktionsprodukt zusammengesetzte homogene Lösung. Als Katalysatoren werden Rhodiumkomplexverbindungen verwendet, die organische Phosphor(III)-Verbindungen als Liganden enthalten. Derartige Komplexverbindungen und ihre Herstellung sind bekannt (zum Beispiel US 3 527 809 A1, US 4 148 830 A1, US 4 247 486 A1, US 4 283 562 A1). Sie können als einheitliche Komplexverbindung oder auch als Gemisch unterschiedlicher Komplexverbindungen eingesetzt werden. Die Rhodiumkonzentration im Reaktionsmedium erstreckt sich über einen Bereich von etwa 1 bis etwa 1000 Gew.-ppm und beträgt vorzugsweise 10 bis 500 Gew.-ppm. Insbesondere wendet man Rhodium in Konzentrationen von 10 bis 200 Gew.-ppm an, jeweils bezogen auf die eingesetzte olefinisch ungesättigte Verbindung. Als Katalysator kann die stöchiometrisch zusammengesetzte Rhodiumkomplexverbindung Anwendung finden. Es hat sich jedoch als zweckmäßig erwiesen, die Hydroformylierung in Gegenwart eines Katalysatorsystems aus der Rhodiumkomplexverbindung und freiem, d.h. überschüssigem Phosphorliganden durchzuführen, der mit Rhodium keine Komplexverbindung mehr eingeht. Der freie Phosphorligand kann der gleiche sein wie in der Rhodiumkomplexverbindung, es können aber auch von diesem verschiedene Liganden eingesetzt werden. Der freie Ligand kann eine einheitliche Verbindung sein oder aus einem Gemisch verschiedene Organophosphorverbindungen bestehen. Beispiele für Rhodiumkomplexverbindungen, die als Katalysatoren Anwendung finden können, sind in US 3 527 809 A1 beschrieben. Zu den bevorzugten Liganden in den Rhodiumkomplexkatalysatoren zählen zum Beispiel Triarylphosphine wie Triphenylphosphin, Trialkylphosphine wie Tri(n-octyl)phosphin, Tri(cyclohexyl)phosphin, Alkylphenylphosphine, Cycloalkylphenylphosphine und organische Phosphite, beispielsweise Tris(2,4-di-t-butylphenyl)phosphit oder die aus WO96/34687 A1 bekannten Bisphosphite. Wegen seiner leichten Zugänglichkeit wird Triphenylphosphin oder Tris(2,4-di-t-butylphenyl)phosphit besonders häufig angewandt.

Üblicherweise beträgt das molare Verhältnis von Rhodium zu Phosphor 1 : 1 bis 1 : 300, jedoch kann der molare Anteil des Phosphors in Form organischer Phosphorverbindungen auch höher sein. Vorzugsweise setzt man Rhodium und organisch gebundenen Phosphor in molaren Verhältnissen von 1 : 3 bis 1 : 200 ein. Bei Anwendung von Triarylphosphinen haben sich insbesondere Rh/P-Molverhältnisse von 1 : 30 bis 1 : 150 bewährt.

Die Hydroformylierungsreaktion wird in Gegenwart eines Lösungsmittels ausgeführt. Als Lösungsmittel werden organische Verbindungen eingesetzt, in denen Ausgangsmaterial, Reaktionsprodukt und Katalysatorsystem löslich sind. Beispiele für solche Verbindungen sind aromatische Kohlenwasserstoffe wie Benzol, Toluol oder die Xylole. Andere gebräuchliche Lösungsmittel sind Paraffinöl, Ketone oder Ether. Als besonders geeignete Lösungsmittel haben sich die hochsiedenden Kondensationsverbindungen der Aldehyde erwiesen, die als hochsiedende Nebenprodukte bei der Hydroformylierungsreaktion entstehen. Der Anteil des Lösungsmittels im Reaktionsmedium kann über einen weiten Konzentrationsbereich variiert werden und beträgt üblicherweise zwischen 10 und 90 Gew.-%, vorzugsweise 10 bis 50 Gew.-%, bezogen auf das Reaktionsgemisch.

Die Hydroformylierungsreaktion wird im Allgemeinen bei einem Druck von 0,5 bis 40 MPa durchgeführt, vorzugsweise von 0,5 bis 10 MPa, aber auch höhere Drücke von 10 bis 40 MPa können angewendet werden. Die Anwendung derartiger hoher Drücke bei der homogen durchgeführten Hydroformylierungsreaktion mit Rhodiumkomplexverbindungen ist aus EP 0 805 138 A1 bekannt.

Die Reaktionstemperatur beträgt im Allgemeinen 50 bis 160°C. Temperaturen von 60 bis 150°C und insbesondere 75 bis 140°C werden bevorzugt.

Das Molverhältnis von Wasserstoff zu Kohlenmonoxid im Synthesegas beträgt 1 : 10 bis 10 : 1, vorzugsweise 1 : 3 bis 3 : 1 und insbesondere 1 : 1. Bei den angegebenen Temperaturen und dem angegebenen Druck ist das im Überschuss zur Hydroformylierung eingesetzte Synthesegas entsprechend seiner Löslichkeit in dem flüssigen Hydroformylierungsaustrag gelöst.

Der flüssige Hydroformylierungsaustrag wird zunächst in einer Entspannungsstufe entspannt, in der eine Auftrennung in eine Flüssigphase und in eine Gasphase erfolgt. Die dabei anfallende Gasphase enthält im Wesentlichen überschüssiges Synthesegas, nicht umgesetzte olefinisch ungesättige Verbindung, hydrierte Produkte und Inertgase aus dem Herstellprozess für Synthesegas. Die Gasphase wird nach Abtrennung eines Teils der Inerten wieder aufkomprimiert und in die Hydroformylierungsreaktion zurückgeführt. Die erhaltene Flüssigphase enthält im Wesentlichen den Aldehyd, hochsiedende Nebenprodukte der Hydroformylierungsreaktion, wie hochsiedende Aldehydkondensationsprodukte, Rhodiumkomplexverbindungen sowie gelöstes Synthesegas. Falls zugesetzt, ist noch ein weiteres organisches Lösungsmittel, wie Toluol oder Xylol zugegen. Vorzugsweise verwendet man jedoch die hochsiedenden Aldehydkondensationsprodukte als Lösungsmittel ohne Zusatz eines weiteren organischen Lösungsmittels. Die Entspannung dient zur Entfernung von in dem flüssigen Austrag gelösten gasförmigen Anteilen und wird daher bei einem Druck durchgeführt, der geringer ist als er Druck in dem Hydroformylierungsreaktor. Der Druck in dem Entspannungsbehälter kann über einen weiten Bereich eingestellt werden. Er kann nur geringfügig geringer sein als der Reaktordruck und im Allgemeinen dem Druckverlust zwischen dem Reaktor und dem nachgeschalteten Entspannungsbehälter entsprechen. Es kann aber auch auf eine tiefere Druckstufe entspannt werden, beispielsweise auf einen Druck in einem Bereich von 0,1 bis 1,5 MPa, vorzugsweise von 0,3 bis 1,0 MPa.

Die im Entspannungsbehälter abgeschiedene Flüssigphase wird anschließend abgeführt und auf eine Trenneinrichtung gegeben, in der der gewünschte Aldehyd von dem homogen gelösten Hydroformylierungskatalysator abgetrennt wird. Dabei wird auf einen Druck entspannt, der niedriger ist als der Druck in dem Entspannungsgefäß. Es bildet sich eine gasförmige Phase, die die Hauptmenge der gewünschten Aldehyde sowie Reste an Synthesegas und leichtsiedenden Verbindungen, beispielsweise durch Hydrierung gebildete Alkane, enthält. Aus der abgeführten Gasphase werden anschließend in weiteren Reinigungsstufen die n- und iso-Aldehyde gewonnen. Die aus der Trenneinrichtung abgeführte Flüssigphase enthält den homogen gelösten Hydroformylierungskatalysator, hochsiedende Aldehydkondensationsprodukte und organisches Lösungsmittel, falls zugesetzt.

Im Allgemeinen wird auf einen Druck entspannt, der bei Normaldruck liegt. Die Trenneinrichtung wird bei einer Temperatur bis 160°C; vorzugsweise von 100 bis 140°C betrieben. Um Schädigungen des Hydroformylierungskatalysators bei seiner Abtrennung von den gewünschten Aldehyden möglichst gering zu halten, darf die Temperatur von 160°C nicht überschritten werden. Die Trenneinrichtung kann in jeglicher Form ausgestaltet werden, die eine thermische Abtrennung des Hydroformylierungskatalysators vom Aldehyd erlaubt. Verwendet werden kann beispielsweise eine Flash-Destillationseinrichtung ohne Einbauten oder eine konventionelle Destillationskolonne, die zur Verbesserung der Trennleistung mit Füllkörpern, wie Raschig-Ringen, Spiralen oder Sattelkörpern, Packungen oder Einbauten, wie Rieselböden ausgestattet sein kann. Die Destillationsbedingungen, wie Temperatur und Druck, richten sich nach den physikalischen Eigenschaften der gewünschten Aldehyde.

Ebenfalls ist es möglich, zwischen dem nach dem Hydroformylierungsreaktor angeordneten Entspannungsbehälter und der zur Aldehydabtrennung von dem Rhodiumkomplexkatalysator dienenden Trenneinrichtung weitere Entspannungsbehälter anzuordnen, in denen von Stufe zu Stufe auf jeweils niedrigere Druckstufen entspannt wird. Diese kaskadenförmige Anordnung ist besonders dann zweckmäßig, wenn die Hydroformylierungsreaktion bei vergleichsweise hohen Drücken durchgeführt wird. Die dabei anfallenden gasförmigen Phasen, die Reste von Synthesegas, nicht umgesetzter olefinisch ungesättigter Verbindung, Inerten und weiteren leichtflüchtigen Komponenten enthalten, werden abgeführt und ausgeschleust oder zweckmäßigerweise teilweise entfernt und nach Komprimierung des Restes wieder in den Hydroformylierungsreaktor zurückgefahren. Die in der jeweiligen Entspannungsstufe erhaltene Flüssigphase, in der der gewünschte Aldehyd und der Hydroformylierungskatalysator in den hochsiedenden Aldehydkondensationsprodukten gelöst vorliegen, wird auf die nächste Entspannungsstufe gegeben. Die dem letzten Entspannungsgefäß entnommene Flüssigphase wird dann in die Trenneinrichtung zur Abtrennung des Aldehyds von dem Hydroformylierungskatalysator geleitet. Für die Stabilisierung des Rhodiumkatalysators ist es zweckmäßig, in der Gasphase des Entspannungsbehälters oder der Entspannungsbehälter einen Kohlenmonoxidpartialdruck von 0,05 bis 0,35 MPa, vorzugsweise von 0,1 bis 0,3 MPa einzustellen. Entsprechend der bei den eingestellten Temperaturen und Drücken herrschenden Löslichkeit liegt Kohlenmonoxid in der flüssigen, organischen Phase gelöst vor und vermag den Rhodiumkomplexkatalysator zu stabilisieren.

Der über den Sumpf der Trenneinrichtung entnommene Flüssigkeitsstrom, der im Wesentlichen aus hochsiedenden Aldehydkondensationsprodukten und organischem Lösungsmittel, falls zugesetzt, besteht und in dem der Rhodiumkomplexkatalysator homogen gelöst vorliegt und der weitere Rhodiumverbindungen von geringerer katalytischer Aktivität teils in suspendierte Form enthält, wird über ein Filter geleitet, um suspendierte Feststoffe abzutrennen. Diese zum Teil rhodiumhaltigen Feststoffe erweisen sich als schädlich, und können nach ihrer Rückführung in die Hydroformylierungszone und in die nachgeschalteten Entspannungsbehälter und Trenneinrichtungen den Rhodiumkomplexkatalysator schädigen und die Abscheidung weiterer rhodiumhaltiger Feststoffe fördern und so zu Rhodiumverlusten beitragen.

Als Filtermedien eignen sich in der Technik übliche Materialien wie Glas- oder Metallfasern oder Kunststofffasern, beispielsweise aus Polypropylen, sofern sie eine ausreichende chemische Resistenz gegenüber der organischen Flüssigkeit aufweisen. Zudem müssen die eingebauten Filtereinrichtungen eine ausreichende mechanische Stabilität gegenüber Beanspruchungen besitzen. Besonders bewährt sind kommerziell erhältliche Filterkerzen oder Glasseidefilterpatronen mit einem Metallkern. Um nicht einen zu hohen Filtrationswiderstand aufzubauen, sollte die Porengröße des Filtermaterials größer als 0,1 µm sein. Hingegen sind zu grobmaschige Materialien aufgrund eines zu geringen Abtrenneffekts ebenfalls ungeeignet. Besonders vorteilhaft sind Filter mit einer Porengröße in einem Bereich von 0,1 bis 20 µm, vorzugsweise von 0,5 bis 10 µm.

Sobald das Filter zu stark belegt ist, wird es ausgebaut. Die darauf niedergeschlagenen Feststoffe, die teilweise Rhodium enthalten, werden separat aufgearbeitet, um das Edelmetall zurückzugewinnen und so den Rhodiumverbrauch zu minimieren.

Ohne zu sehr auf mechanistische Überlegungen eingehen zu wollen, kann man annehmen, dass die in dem zurückgeführten Flüssigkeitsstrom vorhandenen Feststoffe, bei denen es sich um Abrieb aus den Anlagen oder um rhodiumhaltige Desaktivierungs- und Zersetzungsprodukte des Hydroformylierungskatalysators, sogenannte Rhodiumcluster, handelt, als Kristallisationskeime wirken und die Umwandlung der katalytisch aktiven Rhodiumverbindungen in inaktive Feststoffe beschleunigen. Derartige Feststoffe schädigen nicht nur die bereits in der Flüssigphase enthaltenen katalytisch aktiven Rhodiumverbindungen, sondern fördern auch die Verclusterung frisch zugesetzter Rhodiumverbindungen. Frisches Rhodium muss zugesetzt werden, um zum einen die über die hochsiedenden Aldehydkondensationsprodukte ausgeschleusten Rhodiummengen zu ersetzten und zum anderen die Aktivitätsabnahme der im Hydroformylierungsprozess verbleibenden Rhodiumkomplexverbindungen auszugleichen. Durch die erfindungsgemäße Maßnahme, schädliche Feststoffe aus dem in die Hydroformylierungsreaktion zurückgeführten Flüssigkeitsstrom mittels eines Filters zu entfernen, ist pro Kilogramm Einsatz an olefinisch ungesättigter Verbindung weniger frisches Rhodium zuzusetzen, um eine gewünschte Raum-Zeit-Ausbeute an Aldehyden zu erzielen als im Vergleich zu einer Arbeitsweise, bei der ohne Anwendung eines Filters der aus der Trenneinrichtung abgeführte Flüssigkeitsstrom wieder in die Hydroformylierungsreaktion zurückgeführt wird. Arbeitet man nach dem erfindungsgemäßen Verfahren, sind im Allgemeinen 0,5 bis 3 Gew.-ppm frisches Rhodium, bezogen auf eingesetzte olefinisch ungesättigte Verbindung, erforderlich. Demgegenüber steht eine erhöhte Zugabe von frischem Rhodum um zusätzlich 0,5 bis zu 1 Gew.-ppm, bezogen auf eingesetzte olefinisch ungesättigte Verbindung, falls ohne Filter gearbeitet wird. Daraus resultieren dann entsprechend höhere Rhodiumverbräuche.

Der nach dem Durchtritt durch das Filter anfallende flüssige Stoffstrom wird teilweise ausgeschleust, um ein Aufkonzentrieren der Aldehydkondensationsprodukte zu vermeiden. Im stationären Betriebszustand der Hydroformylierungsanlage bleibt die Konzentration an Aldehydkondensationsprodukten konstant, da die während der Hydroformylierungsreaktion gebildete Menge in etwa der ausgeschleusten Menge entspricht. Der nicht ausgeschleuste und von Feststoffen befreite, rhodiumhaltige Flüssigkeitsstrom wird wieder in die Hydroformylierungsreaktion zurückgeführt.

In einer weiteren Ausgestaltung des erfindungsgemäßen Verfahrens wird dem rhodiumhaltigen, flüssigen Sumpfablauf aus der Trenneinrichtung zunächst ein Teilstrom entnommen, der ausgeschleust wird, um ein Aufkonzentrieren der hochsiedenden Aldehydkondensationsprodukte zu vermeiden. Der andere zurückzuführende Teilstrom wird zur Feststoffabtrennung über das Filter geleitet.

Dem in die Hydroformylierungsreaktion zurückgeführten rhodiumhaltigen und von Feststoffen befreiten Flüssigkeitsstrom wird vor dem Eintritt in die Hydroformylierungsreaktion frisches Rhodium und freie organische Phosphor(III)-Verbindung, vorzugsweise die bereits im katalytischen Prozess eingesetzte Phosphorverbindung, zugesetzt. Man setzt die organische Phosphor(III)-Verbindung in einer solchen Menge zu, dass das molare Verhältnis von Rhodium zu Phosphor 1 : 3 bis 1 : 200, vorzugsweise 1 : 30 bis 1 : 150 beträgt.

Frisches Rhodium wird in Form von Rhodiumverbindungen, wie Rhodiumsalzen, beispielsweise Salze aliphatischer Mono- und Polycarbonsäuren, wie Rhodium-2-ethylhexanoat, -acetat, -oxalat, -propionat oder -malonat, Salze anorganischer Wasserstoff- und Sauerstoffsäuren, wie Nitrat, Sulfat oder Chlorid, Rhodiumkomplexverbindungen wie Cyclopentadienylrhodiumverbindungen, Rhodiumacetylacetonat, [RhCl(Cyclooctadien-1,5)]₂, Rhodiumcarbonylverbindungen wie Rh₃(CO)₁₂, Rh₆(CO)₁₆ oder in Form der verschiedenen Rhodiumoxide zugesetzt.

Die für den frischen Rhodiumzusatz verwendete Rhodiumverbindung wird in einem organischen Lösungsmittel gelöst oder, je nach Lösungsverhalten, in dem organischen Lösungsmittel suspendiert. Als zweckmäßige Lösungsmittel haben sich die hochsiedenden Aldehydkondensationsprodukte oder bevorzugt die gewünschten Aldehyde erwiesen.

Nach Zusatz von freier organischer Phosphor(III)-Verbindung und frischem Rhodium, zweckmäßigerweise in Form von Rhodium-2-ethylhexanoat, zu der zurückgeführten, rhodiumhaltigen Lösung wird die so eingestellte Lösung wieder in die Hydroformylierungsreaktion gegeben.

Die Umsetzung kann sowohl absatzweise als auch kontinuierlich durchgeführt werden, wobei die kontinuierliche Prozessführung bevorzugt ist.

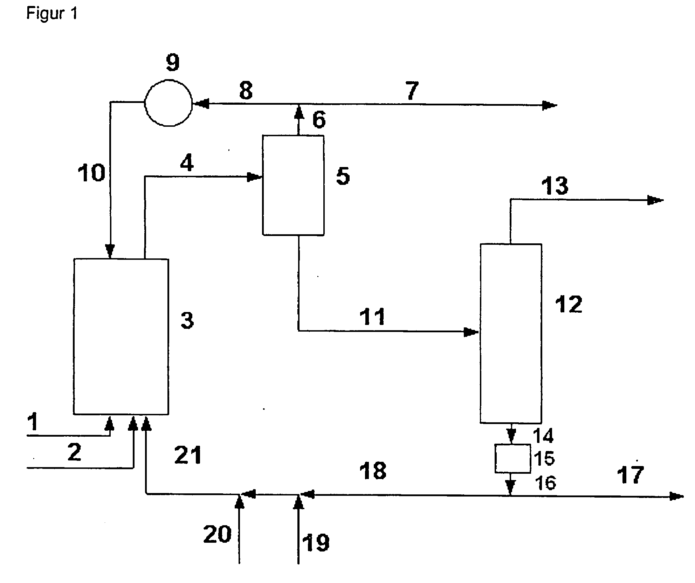

Das bevorzugte erfindungsgemäße Verfahren wird im Folgenden anhand des Prinzipschemas gemäß Figur 1 näher erläutert. Das erfindungsgemäße Verfahren ist aber nicht auf die in der Zeichnung dargestellte Ausführungsform beschränkt.

Über die Leitung (1) wird die olefinisch ungesättigte Verbindung und über die Leitung (2) wird Synthesegas in den Hydroformylierungsreaktor (3) eingeleitet. Der Hydroformylierungsaustrag wird über die Leitung (4) abgezogen und auf den Entspannungsbehälter (5) gegeben. In ihm wird das Reaktionsgemisch auf einen Druck eingestellt, der niedriger ist als der im Hydroformylierungsreaktor (3) herrschende Druck. Es erfolgt eine Trennung in eine Gasphase, die überwiegend Synthesegas, nicht umgesetzte olefinisch ungesättigte Verbindung und leichtflüchtige Verbindungen enthält, und in eine Flüssigphase.

Die Gasphase aus dem Entspannungsbehälter (5) wird über die Leitung (6) abgeführt. Ein Teil wird über die Leitung (7) aus dem Prozess ausgeschleust, um ein Aufkonzentrieren von Inerten zu vermeiden. Der ausgeschleuste Gasstrom kann thermisch verwertet werden. Der nicht ausgeschleuste Gasstrom wird über die Leitung (8) in einen Kompressor (9) geleitet und dort auf Reaktordruck gebracht und über die Leitung (10) in den Hydroformylierungsreaktor (3) zurückgefahren.

Die erhaltene Flüssigphase wird über die Leitung (11) einer Trenneinrichtung (12) zugeführt. Die Flüssigphase enthält im Wesentlichen die Aldehyde, hochsiedende Aldehydkondensationsprodukte als hochsiedende Nebenprodukte der Hydroformylierungsreaktion, den rhodiumhaltigen Hydroformylierungskatalysator, weitere katalytisch weniger aktive oder inaktive Rhodiumkomplexverbindungen und gegebenenfalls Lösungsmittel. In der Trenneinrichtung (12) werden die gewünschten Aldehyde vom Rhodium und Hochsiedern abgetrennt. Bei der Trenneinrichtung (12) handelt es sich vorzugsweise um eine übliche Destillationskolonne, die zum Beispiel mit Füllkörpern, Packungen oder Einbauten für einen intensiven Gas-/Flüssigkeitsaustausch bestückt ist. Die am Kopf der Trenneinrichtung (12) über die Leitung (13) abgezogenen Aldehyde werden anschließend in einer weiteren Destillationsstufe in n- und iso-Aldehyd aufgetrennt (nicht in Figur 1 eingezeichnet). Der rhodiumhaltige Sumpf aus der Trenneinrichtung (12) wird bei einer Sumpftemperatur von höchstens 150°C über die Leitung (14) abgeführt und über das Filter (15), beispielsweise über eine kommerziell erhältliche Filterkerze aus Glasfasern mit einer Porengröße von 0,1 bis 20 µm, geleitet. Dabei werden Feststoffe, beispielsweise Abrieb aus den Anlagenteilen oder während der Hydroformylierungsreaktion, den Entspannungsschritten und der Aldehyd/Katalysatorabtrennung gebildete, in der organischen Flüssigkeit unlösliche Verbindungen zurückgehalten. Es hat sich nämlich gezeigt, dass derartige, in der organischen Lösung vorhandene Feststoffe schädlich wirken und die Desaktivierung und Zersetzung des Rhodiumkomplexkatalysators fördern.

Das von Feststoffen befreite Filtrat fließt über die Leitung (16) ab und wird teilweise über die Leitung (17) aus dem Verfahren entfernt, um ein Aufkonzentrieren der hochsiedenden Aldehydkondensationsprodukte zu vermeiden. Darin enthaltene Rhodiummengen werden extern aufgearbeitet. Der nicht ausgeschleuste Strom wird über die Leitung (18) abgeführt und mit über Leitung (19) herangeführter frischer Rhodiumlösung und mit über Leitung (20) herangeführter Phosphorverbindung versetzt. Die so eingestellte Lösung wird dann über Leitung (21) wieder in den Hydroformylierungsreaktor (3) gegeben. Es ist ebenfalls möglich, die frische Rhodiumlösung mit der Phosphorverbindung vorab zu vermischen und gemeinsam dem rhodiumhaltigen Filtrat zuzusetzen. Sobald das Filter (15) zu stark belegt ist, wird es durch ein frisches Filter ersetzt. Abgeschiedene Feststoffe, die zum Teil Rhodium enthalten, werden separat aufgearbeitet.

Das erfindungsgemäße Verfahren kann auf olefinisch ungesättigte Verbindungen beliebiger Struktur angewandt werden. Dementsprechend sind als Ausgangsmaterial geeignet sowohl Olefine mit innenständiger als auch mit endständiger Doppelbindung und ebenso geradkettige oder verzweigte Olefine. Überdies können die Olefine auch noch durch funktionelle Gruppen substituiert sein, insbesondere solche, die im Verlauf der Reaktion nicht verändert werden. Auch mehrfach olefinisch ungesättigte Verbindungen kommen als Einsatzstoffe in Betracht. Besonders bewährt hat sich das Verfahren bei der Hydroformylierung olefinisch ungesättigter Kohlenwasserstoffe mit 2 bis 25 Kohlenstoffatomen im Molekül, vorzugsweise Ethylen, Propylen, Buten-1, Gemische enthaltend Buten-1 und Buten-2, Hexen-1, Octen-1, dimere Butene, Trimerpropylen oder technisch verfügbare Olefingemische wie Dimersol® oder Octol®.

Die Erfindung wird in dem nachfolgenden Beispiel näher erläutert, jedoch nicht auf die beschriebenen Ausführungsformen beschränkt.

### Beispiel

Propylen wurde mit einem aus gleichen Volumenteilen bestehendes CO/H2-Gemisch bei einem Druck von 5 MPa und einer Reaktionstemperatur von 132°C umgesetzt. Der Katalysator bestand aus hochsiedenden Aldehydkondensationsprodukten, in denen Triphenylphosphin (TPP) und die Rhodiumkomplexverbindung HRhCO (TPP)₃ gelöst sind. Das molare Verhältnis von Rhodium zu Phosphor betrug 1:80, die Rhodiumkonzentration lag bei 100 ppm, bezogen auf eingesetztes Propylen,

Der den Reaktor verlassende Produktstrom wurde zunächst in einen Entspannungsbehälter auf einen Druck von 1,0 MPa entspannt. Die in dem Entspannungsbehälter anfallende Gasphase wurde abgeführt. Ein Teil des abgeführten Gasstromes wurde aus dem Prozess ausgeschleust, der andere Teil nach Aufkomprimierung wieder in den Reaktor zurückgeführt.

Die bei der Entspannung erhaltene flüssige Phase wurde auf eine handelsübliche Destillationskolonne gegeben. Bei einer Sumpftemperatur von 110 bis 130°C und Normaldruck wurde das n/i-Butyraldehydgemisch als Kopfprodukt abgezogen und in einer weiteren Destillationskolonne in n- und iso-Butyraldehyd aufgespalten. Der ausgeschleuste rhodiumhaltige Destillationssumpf wurde anschließend über eine handelsübliche Glasseidenfilterpatrone der Firma Fuhr mit einem Metallkern und mit einer Porengröße von 0,5 µm geleitet.

Das von Feststoffen befreite Filtrat wurde anschließend in zwei Teilströme im Massenverhältnis von 90:10 aufgeteilt. Den massenmäßig geringeren Teilstrom entnahm man dem Prozess, während der andere Teilstrom als Katalysatorkreislauf mit einer Lösung von frischem Rhodium-2-ethylhexanoat in n-Butyraldehyd versetzt wurde. Getrennt davon gab man frisches Triphenylphosphin auf ein Molverhältnis von Rhodium zu Phosphor von 1 : 80 hinzu. Die so eingestellte rhodiumhaltige Lösung wurde anschließend in den Hydroformylierungsreaktor zurückgeführt.

Durch die Filtration des zurückgeführten, rhodiumhaltigen Sumpfablaufs aus der Destillationskolonne kann überraschenderweise der spezifische Rhodiumverbrauch in der Hydroformylierung von Propylen deutlich reduziert werden. Um eine gewünschte Raum-Zeit-Ausbeute zu erhalten, muss nach dem erfindungsgemäßen Verfahren nur noch 0,5 bis 3 Gew.-ppm frisches Rhodium, bezogen auf Propylen, hinzugesetzt werden.

Arbeitet man zu Vergleichszwecken ohne den Filtrierschritt, so ist eine erhöhte Zugabe von frischem Rhodium um zusätzlich 0,5 bis zu 1 Gew.-ppm, bezogen auf Propylen, erforderlich.

## Patentansprüche

1. Verfahren zur Aufarbeitung eines flüssigen Austrags einer Hydroformylierungsreaktion, der Aldehyd, hochsiedende Nebenprodukte der Hydroformylierungsreaktion, einen homogen gelösten Rhodiumkomplexkatalysator, nicht umgesetzte olefinisch ungesättigte Verbindung, Synthesegas und leichtflüchtige Nebenprodukte enthält, bei dem
a) der flüssige Hydroformylierungsaustrag in einem Entspannungsbehälter entspannt wird, wobei eine Auftrennung in eine Flüssigphase und in eine Gasphase, erfolgt; und
b) die in dem Entspannungsbehälter erhaltene Flüssigphase auf eine Trenneinrichtung gegeben wird, wobei eine Auftrennung erfolgt in eine Flüssigphase, die im Wesentlichen hochsiedende Nebenprodukte der Hydroformylierungsreaktion, den homogen gelösten Rhodiumkomplexkatalysator und geringe Mengen des Aldehyds enthält, und in eine Gasphase, die die überwiegende Menge des Aldehyds enthält; und
c) aus der Trenneinrichtung ein flüssiger, rhodiumhaltiger Austrag abgeführt wird,
**dadurch gekennzeichnet, dass** der aus der Trenneinrichtung abgeführte flüssige, rhodiumhaltige Austrag über ein Filter geleitet wird und dabei abgetrennte Feststoffe aus dem Verfahren entfernt werden, während das erhaltene Filtrat in die Hydroformylierungsreaktion zurückgeführt wird.

2. Verfahren gemäß Ansprüche 1, **dadurch gekennzeichnet**, das ein Teil des aus der Trenneinrichtung abgeführten flüssigen, rhodiumhaltigen Austrags aus dem Verfahren entfernt wird während der andere Teil über das Filter geleitet wird.

3. Verfahren gemäß Anspruch 1 oder 2, **dadurch gekennzeichnet, dass** die Trenneinrichtung bei einer Temperatur bis 160°C, vorzugsweise von 100 bis 140°C betrieben wird.

4. Verfahren gemäß einem oder mehreren der Ansprüche 1 bis 3, **dadurch gekennzeichnet, dass** in der Gasphase des Entspannungsbehälters ein Kohlenmonoxidpartialdruck von 0,05 bis 0,35 MPa, vorzugsweise von 0,1 bis 0,3 MPa eingestellt wird.

5. Verfahren gemäß einem oder mehreren der Ansprüche 1 bis 4, **dadurch gekennzeichnet, dass** mehrere Entspannungsbehälter mit von Stufe zu Stufe niedrigeren Druckstufen betrieben werden.

6. Verfahren gemäß einem oder mehreren der Ansprüche 1 bis 5, **dadurch gekennzeichnet, dass** für das Filter Glasfasern, Metallfasern oder Kunststofffasern verwendet werden.

7. Verfahren gemäß Anspruch 6, **dadurch gekennzeichnet, dass** das Filter eine Porengröße von 0,1 bis 20 µm, vorzugsweise von 0,5 bis 10 µm, aufweist.

8. Verfahren gemäß einem oder mehreren der Ansprüche 1 bis 7, **dadurch gekennzeichnet**, das dem Filtrat vor dem Eintritt in die Hydroformylierungsreaktion frisches Rhodium und freie organische Phosphor(III)-Verbindung zugesetzt werden.

## Claims

1. A process for working up a liquid output from a hydroformylation reaction, which output contains aldehyde, high-boiling by-products of the hydroformylation reaction, a homogeneously dissolved rhodium complex catalyst, unreacted olefinically unsaturated compound, synthesis gas and volatile by-products, in which
a) the liquid hydroformylation output is depressurized in a depressurization vessel, resulting in separation into a liquid phase and a gas phase; and
b) the liquid phase obtained in the depressurization vessel is introduced into a separation apparatus in which separation into a liquid phase containing essentially high-boiling by-products of the hydroformylation reaction, the homogeneously dissolved rhodium complex catalyst and small amounts of the aldehyde and a gas phase containing the predominant amount of the aldehyde is carried out; and
c) a liquid, rhodium-containing output is discharged from the separation apparatus,
**characterized in that** the liquid, rhodium-containing output discharged from the separation apparatus is passed through a filter and solids separated off are removed from the process while the filtrate obtained is recirculated to the hydroformylation reaction.

2. The process as claimed in claim 1, **characterized in that** part of the liquid, rhodium-containing output discharged from the separation apparatus is removed from the process while the other part is passed through the filter.

3. The process as claimed in claim 1 or 2, **characterized in that** the separation apparatus is operated at a temperature of up to 160°C, preferably from 100 to 140°C.

4. The process as claimed in one or more of claims 1 to 3, **characterized in that** a carbon monoxide partial pressure of from 0.05 to 0.35 MPa, preferably from 0.1 to 0.3 MPa, is set in the gas phase in the depressurization vessel.

5. The process as claimed in one or more of claims 1 to 4, **characterized in that** a plurality of depressurization vessels having pressures which decrease from stage to stage are employed.

6. The process as claimed in one or more of claims 1 to 5, **characterized in that** glass fibers, metal fibers or polymer fibers are used for the filter.

7. The process as claimed in claim 6, **characterized in that** the filter has a pore size of from 0.1 to 20 µm, preferably from 0.5 to 10 µm.

8. The process as claimed in one or more of claims 1 to 7, **characterized in that** fresh rhodium and free organic phosphorus(III) compound are added to the filtrate before entry into the hydroformylation reaction.

## Revendications

1. Procédé pour le traitement d'un produit liquide d'une réaction d'hydroformylation, qui contient l'aldéhyde, des produits secondaires de point d'ébullition élevé de la réaction d'hydroformylation, un catalyseur complexe au rhodium dissous en phase homogène, le composé oléfiniquement insaturé non transformé, un gaz de synthèse et des produits secondaires volatils, dans lequel
a) le produit d'hydroformylation liquide est détendu dans un récipient de détente, une séparation en une phase liquide et une phase gazeuse se produisant ; et
b) la phase liquide obtenue dans le récipient de détente est transférée sur un dispositif de séparation, où se produit une séparation en une phase liquide qui contient essentiellement des produits secondaires de point d'ébullition élevé de la réaction d'hydroformylation, le catalyseur complexe au rhodium dissous en phase homogène et de faibles quantités de l'aldéhyde, et en une phase gazeuse qui contient la quantité principale de l'aldéhyde ; et
c) un produit liquide, contenant du rhodium est évacué du dispositif de séparation,
**caractérisé en ce que** le produit liquide, contenant du rhodium, évacué du dispositif de séparation est guidé sur un filtre et les solides séparés sont ainsi séparés du procédé, alors que le filtrat obtenu est recyclé dans la réaction d'hydroformylation.

2. Procédé selon la revendication 1, **caractérisé en ce qu'**une partie du produit liquide, contenant du rhodium, évacué du dispositif de séparation est éliminée du procédé alors que l'autre partie est guidée sur le filtre.

3. Procédé selon la revendication 1 ou 2, **caractérisé en ce que** le dispositif de séparation est exploité à une température de jusqu'à 160°C, de préférence de 100 à 140°C.

4. Procédé selon l'une ou plusieurs des revendications 1 à 3, **caractérisé en ce qu'**on règle dans la phase gazeuse du récipient de détente une pression partielle en monoxyde de carbone de 0,05 à 0,35 MPa, de préférence de 0,1 à 0,3 MPa.

5. Procédé selon l'une ou plusieurs des revendications 1 à 4, **caractérisé en ce que** plusieurs récipients de détente, présentant des niveaux de pression diminuant d'étage en étage, sont exploités.

6. Procédé selon l'une ou plusieurs des revendications 1 à 5, **caractérisé en ce qu'**on utilise, pour le filtre, des fibres de verre, des fibres métalliques ou des fibres en matériau synthétique.

7. Procédé selon la revendication 6, **caractérisé en ce que** le filtre présente une taille de pores de 0,1 à 20 µm, de préférence de 0,5 à 10 µm.

8. Procédé selon l'une ou plusieurs des revendications 1 à 7, **caractérisé en ce que** le filtrat est additionné, avant l'entrée dans la réaction d'hydroformylation, de rhodium frais et de composé organique libre de phosphore (III).
